Europäisches Patentamt

**European Patent Office**

Office européen des brevets ·

(11) Veröffentlichungsnummer : **0 007 521**
**B1**

(12) **EUROPÄISCHE PATENTSCHRIFT**

(45) Veröffentlichungstag der Patentschrift :
**13.01.82**

(21) Anmeldenummer : **79102382.3**

(22) Anmeldetag : **11.07.79**

(51) Int. Cl.³ : **G 01 N 33/18, G 01 N 31/12**

(54) Verfahren zum quantitativen Bestimmen des Sauerstoffbedarfs von oxidierbare Stoffe enthaltendem Wasser.

(30) Priorität : **21.07.78 DE 2832043**

(43) Veröffentlichungstag der Anmeldung :
**06.02.80 (Patentblatt 80/03)**

(45) Bekanntmachung des Hinweises auf die Patenterteilung : **13.01.82 Patentblatt 82/02**

(84) Benannte Vertragsstaaten :
**AT BE CH DE FR GB IT NL SE**

(56) Entgegenhaltungen :
**DE - A - 2 220 976**
**DE - A - 2 427 046**
**US - A - 3 933 429**
**US - A - 3 679 364**

(73) Patentinhaber : **HOECHST AKTIENGESELLSCHAFT**
**Postfach 80 03 20**
**D-6230 Frankfurt/Main 80 (DE)**

(72) Erfinder : **Melzer, Werner, Dr.**
**Drosselweg 2**
**D-6237 Liederbach (DE)**
Erfinder : **Jaenicke, Dieter**
**Am Heigenstock 10**
**D-6238 Hofheim am Taunus (DE)**

Imprimerie Jouve, 18, rue St-Denis, 75001 Paris, France

Verfahren zum quantitativen Bestimmen des Sauerstoffbedarfs von oxidierbare Stoffe enthaltendem Wasser

Die Erfindung bezieht sich auf ein Verfahren zum quantitativen Bestimmen des Sauerstoffbedarfs von oxidierbare Stoffe enthaltendem Wasser durch kontinuierliches Verdampfen des Wassers in Gegenwart von Sauerstoff bei ca. 900 °C.

Im Folgenden bedeutet :

TOD (Total Oxygen Demand) der gesamte Sauerstoffbedarf von oxidierbare Stoffe enthaltendem Wasser ;

sauerstoffhaltiges Gas reiner Sauerstoff und/ oder alle sauerstoffhaltigen Gase, insbesondere Luft.

Nach einem bekannten Verfahren (vergl. z.B. US-A-3679364) zum Bestimmen des TOD-Wertes wird oxidierbare Stoffe enthaltendes Wasser in kleinen Mengen in einen auf ca. 900 °C erwärmten und im Vergleich zur Wassermenge großen und konstanten sauerstoffhaltigen Trägergasstrom dosiert und verdampft. Das Volumenverhältnis Wasserdampf zu Trägergas beträgt etwa 1 : 100. Etwa im Wasser vorhandene oxidierbare Stoffe werden dabei oxidiert. Danach wird die Konzentrationsabnahme des Sauerstoffs im Vergleich zum unveränderten Trägergas gemessen und aus dem Meßwert der TOD-Wert bestimmt. Nachteilig bei dieser Methode sind die hohen Anforderungen an die Dosiergenauigkeit der Wasserprobe, insbesondere bei stark verschmutztem Abwasser.

Die Aufgabe bestand demnach darin, ein Verfahren zu schaffen, bei dem die Anforderungen an die Dosiergenauigkeit sehr gering sind.

Die Erfindung löst die Aufgabe dadurch, daß

a) dem Wasserdampf taktkontinuierlich unterschiedliche Mengen $l_1,....,l_n$ sauerstoffhaltigen Gases pro Mengeneinheit Wasserdampf zugeführt werden, wobei die jeweilige Menge sauerstoffhaltigen Gases 1-5 Volumenprozent, bezogen auf die während des jeweiligen Taktes erzeugte Wasserdampfmenge nicht überschreiten soll,

b) die zu $l_1,....,l_n$ sich jeweils einstellenden Restkonzentrationen $S_1$-$S_n$ an Sauerstoff gemessen werden und

c) aus den Restkonzentrationen $S_1,....,S_n$ und den in Schritt a) zugeführten Mengen $l_1,....,l_n$ sauerstoffhaltigen Gases der Sauerstoffbedarf TOD ermittelt wird.

Der Sauerstoffbedarf (TOD) läßt sich aus den gemessenen Restkonzentrationen $S_1,....,S_n$ und den zugeführten Mengen an sauerstoffhaltigem Gas $l_1,....,l_n$ dadurch ermitteln, daß man in einem Diagramm als Abszisse die Mengen $l_1,....,l_n$ und auf der Ordinate die zugehörigen Restkonzentrationen $S_1,....,S_n$ aufträgt, die so erhaltenen Punkte durch eine Kurve verbindet wobei der Schnittpunkt dieser Kurve mit der Ordinate die Abnahme der Sauerstoffkonzentrationen bezogen auf die Ausgangskonzentration ergibt. Die Abnahme ist dem TOD-Wert direkt proportional.

Der TOD-Wert läßt sich auch rechnerisch aus der Gleichung

$$TOD = \alpha \frac{1}{n} \sum \frac{S_1 l_n - S_n \cdot l_1}{l_n - l_1} \; ; \; n = 2, 3,...$$

bestimmen.

Sofern man dafür sorgt, daß der Taupunkt nicht unterschritten wird, kann der sauerstoffhaltige Gase enthaltende Wasserdampf direkt in den Sauerstoffanalysator geleitet werden. Der sauerstoffhaltige Gase enthaltende Wasserdampf kann jedoch auch in einen konstanten sauerstofffreien Trägergasstrom dosiert werden. Vor Einleiten dieses Trägergasstromes in den Sauerstoffanalysator ist jedoch der Wasserdampf, z.B. durch Kondensation, zu entfernen. Bei dieser Methode sollte das Volumenverhältnis Wasserdampf zu Trägergas möglichst konstant gehalten werden.

Mit dem erfindungsgemäßen Verfahren läßt sich besonders gut der Gehalt oxidierbarer Stoffe in Abwasser überwachen. Hervorzuheben ist ferner die große Unempfindlichkeit gegen Dosierschwankungen der Wasserprobe. So ergeben sich bei einem Volumenverhältnis Wasserdampf zu Luft von 100 : 1 bei einer Schwankung der Wasserdosierung um den Faktor 2 nur Fehler von 2 % bezogen auf den Meßwert der Restkonzentration an Sauerstoff im Gasgemisch.

Im Folgenden wird die Erfindung anhand der Figuren näher erläutert. Im Reaktionsgefäß 2, dem über Leitung 1 kontinuierlich Wasser und über Leitung 3 mit Dosiervorrichtung 8 umschaltbare Mengen Luft zugeführt wird, wird das Wasser bei ca. 900 °C verdampft und die im Wasser vorhandenen oxidierbaren Stoffe umgesetzt. Die Sauerstoffmenge des sauerstoffhaltigen Gases soll so bemessen sein, daß sie für die Oxidierung der oxidierbaren Stoffe im Wasser ausreicht. Sauerstoffmengen von 1-5 Vol.-% bezogen auf das Wasserdampfvolumen, haben sich als ausreichend erwiesen. Der das Reaktionsgefäß 2 verlassende sauerstoffhaltige Gase enthaltende Wasserdampf wird einem Sauerstoffanalysator 4 (z.B. Zirkonoxid-Meßzelle) zugeführt und die Restkonzentration an Sauerstoff in diesem Gemisch gemessen.

In einer Verfahrensvariante wird der das sauerstoffhaltige Gas enthaltende Wasserdampf über eine Dosiervorrichtung 5 in einen konstanten sauerstofffreien Trägergasstrom 7 z.B. im Verhältnis 1 : 1 dosiert. In Einrichtung 6 wird der Wasserdampf aus dem Gemisch aus Trägergas und sauerstoffhaltigem Gas enthaltendem Wasserdampf z.B. durch Trocknung oder Kondensation entfernt und anschließend der Sauerstoffgehalt im Gas festgestellt. Bei dieser Verfahrensvariante ist es außerdem erforderlich, eine Kondensation von Wasserdampf auf dem Weg von Reaktionsgefäß 2 zur Einrichtung 6 zu vermeiden.

Vorrichtung 9 dient dazu, die Luftdosierung 8 umzuschalten, die zu den unterschiedlich dosierten Luftmengen gehörende Restkonzentration an Sauerstoff zeitlich richtig zu verknüpfen und den TOD-Wert zu bestimmen.

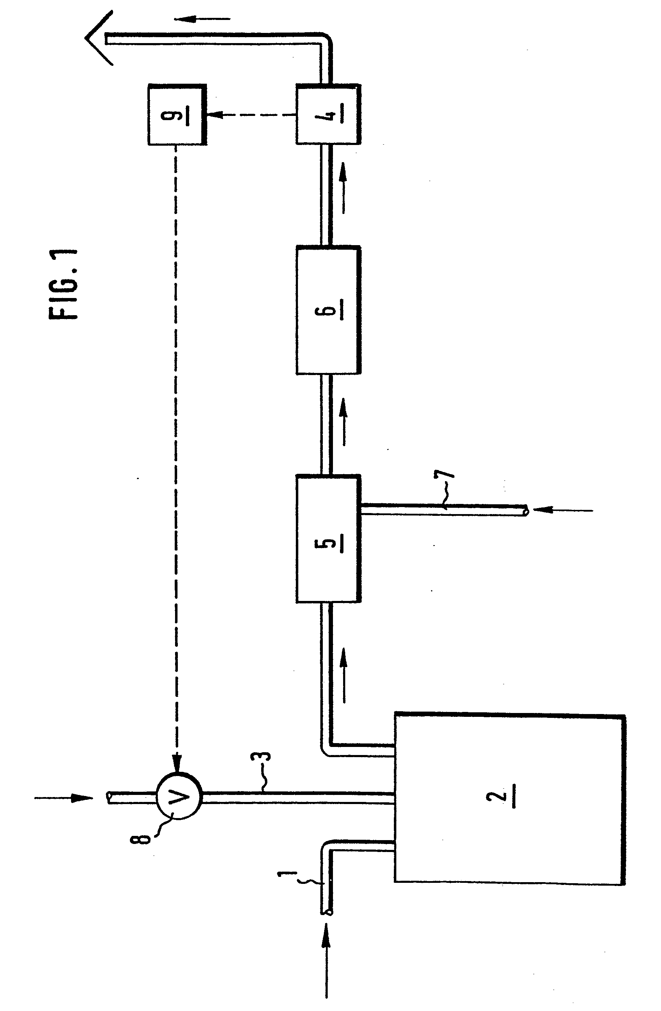

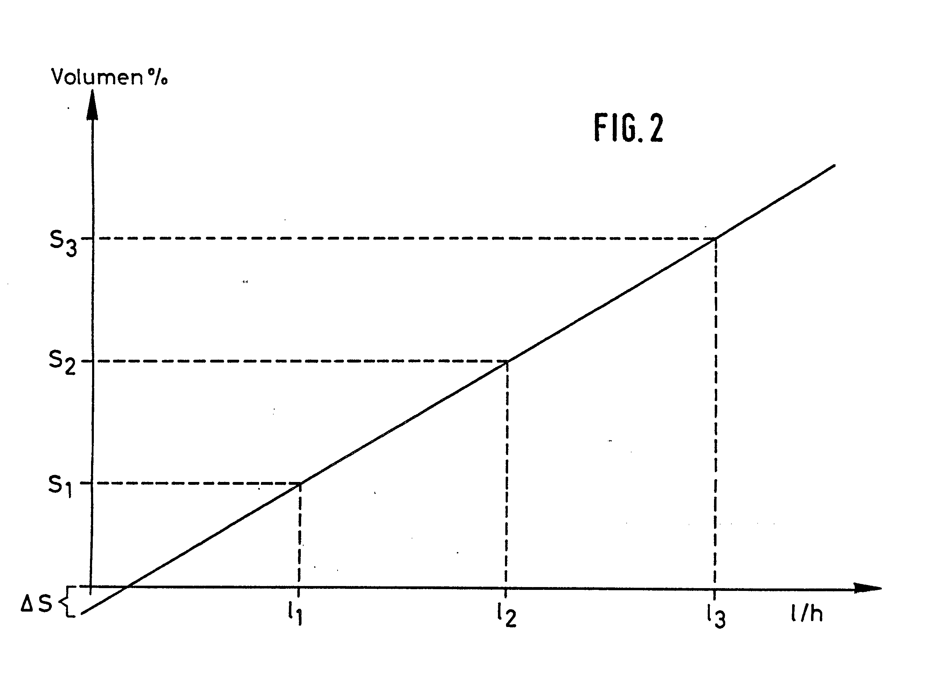

In Fig. 2 sind auf der Abszisse die Menge $I_1$ ... $I_n$ sauerstoffhaltigen Gases in l/h aufgetragen, auf die Ordinate die zugehörigen sich jeweils einstellenden Restkonzentrationen $S_1$, ..., $S_n$ an Sauerstoff in Vol.-%. $\Delta S$ entspricht der Abnahme der Sauerstoffkonzentration im Ausgangsgas hervorgerufen durch die Verunreinigung des Wassers. $\Delta S$ ist dem TOD-Wert direkt proportional.

Beispiel

In den etwa 200 ml Volumen fassenden Reaktionen wurde kontinuierlich mittels Förderpumpe 100 g H₂O/h gefördert, welches mit 1,28 g Methanol/l (entsprechend einem TOD-Wert von 1,92 g O₂/l) verunreinigt war. In den Reaktor wurde zunächst ein Sauerstoffvolumensstrom $I_1 = 0,5$ l/h und dann ein Volumensstrom $I_2 = 1$ l/h eingespeist und mit einem auf über + 100 °C aufgeheiztem Sauerstoffmeßgerät die zugehörigen Restkonzentrationen an Sauerstoff $S_1 = 0,30$ Vol.-% und $S_2 = 0,70$ Vol.-% ermittelt. Die durch die Wasserverunreinigung bedingte Abnahme der Sauerstoffkonzentration ergibt sich somit zu

$$\Delta S = \frac{S_1 I_2 - S_2 I_1}{I_2 - I_1} = \frac{0,3 \cdot 1 - 0,7 \cdot 0,5}{1 - 0,5} = -0,1 \text{ Vol.-\%}$$

und entspricht einem TOD-Wert von 1,92 gO₂/l.

**Ansprüche**

1. Verfahren zum quantitativen Bestimmen des Sauerstoffbedarfs (TOD) von oxidierbare Stoffe enthaltendem Wasser durch kontinuierliches Verdampfen des Wassers in Gegenwart von Sauerstoff bei ca. 900 °C, dadurch gekennzeichnet, daß

a) dem Wasserdampf taktkontinuierlich unterschiedliche Mengen $I_1$, ..., $I_n$ sauerstoffhaltigen Gases pro Mengeneinheit Wasserdampf zugeführt werden, wobei die jeweilige Sauerstoffmenge im sauerstoffhaltigen Gas 1-5 Vol.-%, bezogen auf die während des jeweiligen Taktes erzeugte Wasserdampfmenge nicht überschreiten soll,

b) die zu $I_1$, ..., $I_n$ sich jeweils einstellenden Restkonzentrationen $S_1$, ..., $S_n$ an Sauerstoff im Wasserdampf/Gasgemisch gemessen werden und

c) aus den Restkonzentrationen $S_1$, ..., $S_n$ und den in Schritt a) zugeführten Mengen $I_1$, ..., $I_n$ sauerstoffhaltigen Gases der Sauerstoffbedarf (TOD) ermittelt wird.

2. Verfahren nach Anspruch 1, dadurch gekennzeichnet, daß das Gemisch aus sauerstoffhaltigem Gas und Wasserdampf aus Schritt a) in einen mengenmäßig konstanten sauerstofffreien Trägergasstrom eingeleitet, danach der Wasserdampf entfernt und die Schritte b) und c) durchgeführt werden.

**Claims**

1. Process for the quantitative determination of the oxygen demand (TOD) of water containing oxidizable matter by continously evaporating the water at about 900 °C in the presence of oxygen, which comprises

a) adding to the steam at continously repeated intervals varying amounts $I_1$, ..., $I_n$ of oxygen-containing gas per unit of steam, the respective amount of the oxygen-containing gas not exceeding 1 to 5 % by volume, calculated on the amount of steam produced during the corresponding interval ;

b) measuring the residual oxygen concentration $S_1$-$S_n$ for each amount of oxygen-containing gas $I_1$, ..., $I_n$ and

c) calculating the TOD from the residual concentrations $S_1$, ..., $S_n$ and the amounts of oxygen-containing gas $I_1$, ..., $I_n$ introduced in step a).

2. The process as claimed in claim 1, wherein the mixture of oxygen-containing gas and steam of step a) is introduced into a constant flow of oxygen-free carrier gas, the steam is removed, whereupon steps b) and c) are carried out.

**Revendications**

1. Procédé pour déterminer quantitativement la demande d'oxygène (DTO) d'une eau contenant des substances oxydables, par évaporation continue de l'eau en présence d'oxygène à 900 °C environ, procédé caractérisé en ce que

a) on introduit dans la vapeur d'eau, à une cadence continue, des quantités différentes $I_1$, ..., $I_n$ de gaz contenant de l'oxygène par quantité unitaire de vapeur d'eau, chaque quantité de gaz contenant de l'oxygène ne devant pas dépasser 1 à 5 % en volume, par rapport à la quantité de vapeur d'eau formée pendant la période respective,

b) on mesure les concentrations résiduelles en oxygène $S_1$, ..., $S_n$ s'établissant à chaque fois pour $I_1$, ..., $I_n$, et

c) on détermine, à partir des concentrations résiduelles $S_1$, ..., $S_n$ et des quantités $I_1$, ..., $I_n$ de gaz contenant de l'oxygène introduites au stade a), la demande totale en oxygène DTO.

2. Procédé selon la revendication 1, caractérisé en ce qu'on introduit le mélange de gaz contenant de l'oxygène et de vapeur d'eau provenant du stade a) dans un courant de gaz porteur exempt d'oxygène dont la quantité est constante, après quoi on élimine la vapeur d'eau et on effectue les opérations b) et c).

FIG. 1

FIG.2

Volumen%

$S_3$

$S_2$

$S_1$

$\Delta S$

$l_1$  $l_2$  $l_3$  $l/h$